# EUROPÄISCHE PATENTSCHRIFT

(11) **EP 1 131 053 B1**
(45) Veröffentlichungstag und Bekanntmachung des Hinweises auf die Patenterteilung: **27.09.2006**
(21) Anmeldenummer: 99966943.5
(22) Anmeldetag: 15.11.1999
(51) Int. Cl.: A61K 9/127, A61K 9/50

(54) **NANOKAPSELN UND VERFAHREN ZUR HERSTELLUNG DIESER**
NANOCAPSULES AND METHOD OF PRODUCTION THEREOF
NANOCAPSULES ET LEUR PROCEDE DE PRODUCTION

(30) Priorität: 17.11.1998 DE 19852928
(43) Veröffentlichungstag der Anmeldung: 12.09.2001
(73) Patentinhaber: novosom AG, 06120 Halle (DE)
(72) Erfinder: PANZNER, Steffen, D-06108 Halle / Saale (DE)
(74) Vertreter: Ziebig, Marlene
(86) Internationale Anmeldenummer: PCT/EP1999/009744
(87) Internationale Veröffentlichungsnummer: WO 2000/028972

(56) Entgegenhaltungen:
- EP-A- 0 199 362
- US-A- 5 834 556

## Beschreibung

Die Erfindung betrifft Nanokapseln mit einer Größe zwischen 50 nm und 10 µm Durchmesser, deren Hüllschicht aus mindestens zwei verschiedenen, miteinander vernetzten Polymeren P1 und P2 besteht, wobei gegebenenfalls unter dieser Hüllschicht noch eine Lipidschicht vorhanden ist. Die erfindungsgemäßen Nanokapseln werden durch kovalente Vernetzung von mindestens zwei verschiedenen wasserlöslichen Polymeren P1 und P2 auf der Oberfläche von Liposomen hergestellt, wobei die Liposomen nach der Vernetzung gegebenenfalls aufgelöst werden. Die erfindungsgemäßen Nanokapseln können biologisch aktive Verbindungen tragen.

Nanokapseln oder Nanokugeln sind partikuläre Strukturen im Größenbereich zwischen 50 nm und 10 µm, bei denen eine Hüllschicht einen Binnenraum vom Außenmedium abgrenzt. Diese Eigenschaft unterscheidet Nanokapseln von Nanosphären; die letzteren besitzen einen einheitlichen Querschnitt. Strukturen mit gleichem Aufbau sind auch in größeren Dimensionen bekannt und werden dann als Mikrokapseln bezeichnet. Liposomen und Viruskapside sind weitere verwandte Strukturen der Nanokapseln.

Zur Herstellung von Nanokapseln lassen sich vorteilhaft Verfahren einsetzen, bei denen Vernetzungsreaktionen an Phasengrenzflächen ausgeführt werden. Der mögliche Nutzen solcher Partikel ist maßgeblich von der verwendeten Hüllschicht und dem Herstellungsverfahren abhängig. Bekannte Hüllschichten nach dem Stand der Technik sind solche aus vernetzten Proteinen oder Grenzflächenpolymerisate, insbesondere aus Derivaten der Acrylsäure.

Hüllschichten , die ganz oder teilweise aus Proteinen bestehen sind von besonderem Interesse, da sie biokompatibel und abbaubar ausgeführt werden können. Die zum Aufbau verwendeten Proteine sind strukturbildend, können aber auch aktivitätstragend sein. Solche Partikel sind zum Einschluß von Fremdstoffen sowie zur Bindung von anderen Komponenten an die Oberfläche geeignet. Aufgrund der natürlichen Vielfalt der einsetzbaren Proteine sind die Oberflächeneigenschaften in hohem Maße variabel und können unterschiedlichen Anforderungen angepaßt werden.

Membranen aus Surface-layer (S-layer)-Proteinen wurden (im EP 0154 620) beschrieben. Diese Membranen entstehen durch Rekristallisation der S-layer-Proteine in freier Lösung oder an der Oberfläche von Liposomen.
Im letzteren Fall ist der vorherige Einschluß von Makromolekülen möglich, die Liposomen werden durch das Aufbringen der Membran wesentlich stabilisiert (Kupcu, S., Sara, M. und Sleytr, U.B., Biochem. Biophys. Acta, 1235 (2): 263-269 (1995)). Bei flächig-kristallinem

Aufbau der Membranen ergeben sich Strukturen mit einer regelmäßigen Anordnung von gleichartigen Poren, die vorteilhaft bei der Ultrafiltration verwendet werden können.
Die ebenfalls regelmäßige Anordnung chemischer Gruppen auf der Oberfläche führt bei der Bindung von anderen Makromolekülen zu einer sehr homogenen Verteilung, die vorteilhaft bei der Anwendung in Detektionssystemen ist. Limitierend für den Einsatz in biologischen Systemen kann sich bei den Membranen der S-layer-Proteinen deren Immunogenität auswirken. S-layer-Proteine erzeugen eine starke Immunantwort und werden daher als Adjuvans verwendet (US 5 043 158). Zudem sind die S-layer-Proteine nicht selbst aktivitätstragend.

Die US 5,834,556 beschreibt ein Verfahren zur Herstellung von Nanokapseln mit einem Durchmesser von 500 nm-560 nm. Das Verfahren offenbart weiterhin die Beschichtung der Liposomen mit Graftcopolymeren. Bei dem Verfahren gemäß der US 5, 834, 556 wird zunächst ein Copolymer hergestellt, mit dem in einem zweiten Schritt Liposomen beschichtet werden.

Die US-Patente US 5,498,421; US 5,635,207; US 5,650,156; US 5,665,383; US 5,639,473 sowie US 5,512,268 beschreiben die Herstellung und Nutzung von Hohlkugeln im Größenbereich bis 10*µ*m, bei denen an der Phasengrenze zu einem nicht wassermischbaren Kern eine Hüllschicht ausgebildet wird. Diese Hüllschicht wird durch Disulfidbrücken stabilisiert und kann aus Proteinen, insbesondere Hämoglobin oder Albumin oder anderen thiolhaltigen Polymeren gebildet werden. Die Emulgierung der nichtmischbaren Phase wird durch starken Ultraschall bewirkt. Bei diesem Vorgang bildet sich unter anderem Wasserstoffperoxid, dass zu einer oxidativen Vernetzung der Hüllkomponenten führt.

Aus Hämoglobin hergestellte Partikel sind zur Aufnahme und Abgabe von Sauerstoff fähig, allerdings mit anderem Hill-Koeffizienten als natürliches Hämoglobin. Sie lassen sich als Blutersatz verwenden.
In anderen Verwendungen werden Gase oder Kontrastmittel in die Partikel eingeschlossen und bei bildgebenden Verfahren in der Medizin eingesetzt. In wieder anderen Verwendungen wird die Verpackung biologisch wirksamer Substanzen beschrieben, insofern diese ohne Verlust ihrer Aktivität in der inneren Phase gelöst oder emuligiert werden können. Für eine Verpackung von hydrophilen Makromolekülen wie Proteinen oder Nukleinsäuren ist das Verfahren daher nur bedingt geeignet.

Weitere Hohlkugeln im Nanometerbereich lassen sich durch wiederholte Abscheidung von Polyelektrolyten auf kolloidal gelösten Partikeln darstellen (Caruso, F.; Caruso, R.A. und Möhwald, H. (1998) Science 282:1111-1113). Im Beispiel werden sehr kleine Silicapartikel im Wechsel mit Poly(diallyldimethylammoniumchlorid) auf einer Polystyrenmatrix abgeschieden. Diese Matrix kann anschließend durch Kalzinierung oder Lösungsmittel entfernt werden, so dass die Hohlkugeln zurückbleiben.

Es ist bekannt, Liposomen und Nanokapseln zum Einschluß von biologisch aktiven Verbindungen einzusetzen, etwa bei pharmazeutischen Formulierungen. Sie können ihr Cargo an einen Wirkort bringen oder dieses über einen längeren Zeitraum freisetzen. Die umgebende Membran kann den eingeschlossenen Wirkstoff vor Abbau oder Inaktivierung schützen.

Die Natur des eingeschlossenen Wirkstoffes, insbesonere seine Löslichkeit und sein Molekulargewicht sind in weiten Grenzen variierbar. Liposomen sind darüberhinaus wegen ihrer immunologischen Verträglichkeit besonders geeignete Systeme für die Verpackung pharmazeutischer Wirkstoffe.
Besonders gestaltete Liposomen können zur Einbringung von Nukleinsäuren in Säugetierzellen benutzt werden. In einer vorteilhaften Variante dieser Technik werden Lipid-Nukleinsäure-Komplexe unter der Verwendung kationischer Lipide erzeugt und die zu behandelnden Zellen damit transfiziert. Die Transfektion ist einfach, aber wenig effektiv un unspezifisch.
In einer andern Ausgestaltung werden pH-sensitive Liposomen auf dem endozytotischen Weg von den Zielzellen aufgenommen. Im sauren Kompartiment der Endosomen fusionieren diese mit der umgebenden Membran und bringen auf diesem Wege ihr Cargo in das Zellinnere. Mit diesem Verfahren lassen sich auch Proteine und andere Wirkstoffe in das Zellinnere transportieren.

Liposomen können auch als Detektionssysteme mit hoher Signalverstärkung verwendet werden (US 4 622 294). Die Signalverstärkung wird in diesem Fall durch die hohe Anzahl von eingeschlossenen Enzymmolekülen im Verhältis zur detektierten Spezies erreicht. Nachteilig bei der Verwendung von konventionellen Liposomen ist deren Empfindlichkeit gegenüber Detergenzien, die bei veranden Anwendungen zur Unterdrückung unspezifischer Wechselwirkungen eingesetzt werden.

Die bekannten Hohlkugeln haben folgende Nachteile:

Die Hohlkugeln, die auf der Verwendung von S-layern beruhen, weisen einen wäßrigen Binnenraum und eine definierte Permeabilität der Hüllschicht auf. Die Möglichkeit zur Verpackung hydrophiler Makromoleküle ist gegeben. Nachteilig ist aber die Beschränkung der nutzbaren Verbindungen auf die S-layer-Proteine. Diese sind nicht aktivitätstragend und zeigen antigene Wirkung.
Beim Verfahren nach US 5 498 421 und den anderen genannten US-Schriften entsteht eine funktionelle Hüllschicht aus Proteinen in einer Phasengrenze. Das System ist nur bedingt für den Einschluß von hydrophilen Makromolekülen geeignet. Die Komponenten der Hüllschicht sind sehr starr miteinander vernetzt, daraus resultiert eine Veränderung der Eigenschaften beim verwendeten Hämoglobin. Die nutzbaren Komponenten zum Aufbau der Hülle sind auf solche Polymere beschränkt, die eine Vielzahl von Thiolfunktionen besitzen und sich an den verwendeten Phasengrenzflächen anlagern.
Nachteilig bei konventionellen liposomalen Systemen ist ihre geringe mechanische und in-vivo Stabilität. Die Partikel werden innerhalb kurzer Zeit von Makrophagen des retikuloendothelialen Systems aufgenommen und somit aus der Zirkulation entfernt.

Aufgabe der Erfindung war es deshalb, Nanokapseln bereitzustellen, die die genannten Nachteile nicht aufweisen.

Erfindungsgemäß gelingt dies durch Nanokapseln mit einer Größe zwischen 50 nm und 10 µm im Durchmesser, die durch kovalente Vernetzung von zwei verschiedenen wasserlöslichen Polymeren P1 und P2, die eine größere Anzahl funktioneller Gruppen aufweisen, auf der Oberläche von Liposomen hergestellt wurden. Das Verfahren zur Herstellung der erfindungsgemäßen Nanokapseln ist dadurch gekennzeichnet, daß zunächst Liposomen hergestellt werden, diese mit einem Polymer P1 beschichtet werden, indem das Polymer P1 in wäßriger Lösung an die Liposomenoberfläche gebunden wird und dann das aufgebrachte Polymer P1 mit einem von P1 verschiedenen Polymer P2 in wäßriger Lösung kovalent vernetzt wird und gegebenenfalls noch weitere Polymerschichten durch Vernetzung aufgebracht werden.

Als Ausgangsmaterial werden Liposomen verwendet, deren Größe die der entstehenden Nanokapseln bestimmt. Geeignete Methoden zur Herstellung solcher Liposomen sind an sich bekannt.

Eine vorteilhafte Variante der Herstellung von Liposomen umfaßt die Auflösung der Membranbestandteile in Ethanol und die Mischung der Lösung mit Wasser oder wäßrigen Pufferlösungen. Aus den so gewonnenen multilamellaren Liposomen werden durch Behandlung im Hochdruckhomogenisator (French Press) kleinere uni- oder oligolamellare Vesikel mit enger Größenverteilung hergestellt.
Eine Variation dieser Technik ist die Passage der multilamellaren Ausgangsliposomen durch isopore Membranen, die ebenfalls zur Entstehung von uni- und oligolamellaren Liposomen mit enger Größenverteilung führt.
Nach einem andern Verfahren werden uni- und oligolamellare Liposomen aus einer Detergens-Lipid Phase durch Entfernung des Detergens hergestellt. Das kann durch Gelfiltration oder Dialyse erreicht werden.

Erfindungsgemäß müssen die verwendeten Liposomen die Bindung des wasserlöslichen Polymers P1 ermöglichen. Methoden für die kovalente Kopplung in wäßrigen Medien sind dem Fachmann bekannt (G.Hermanson, Bioconjugate Techniques, Academic Press 1996) und beinhalten die heterofunktionelle oder homofunktionelle Verknüpfung von Amino-, Thiol-, Hydrazo-, Hydroxy-, Azidwasserstoff-, Aldehyd-, Carboxylgruppen oder von deren aktivierten Estern in geeigneten Kombinationen.

Liposomen können solche funktionellen Gruppen enthalten. Alternativ können durch chemische Modifiation der Lipidbestandteile solche Gruppen auf der Oberfläche von Liposomen erzeugt werden.
Zu den geeigneten membranbildenden oder membranständigen Verbindungen mit solchen Gruppen gehören unter anderem: Phosphatidylethanolamin, Phosphatidylserin, Phosphatidylglycerol, Phosphatidylinositol und die Derivate dieser Verbindungen, insbesondere solche mit freier Thiol-, Amino-, Carboxyl-, Aktivester- oder Aldehydfunktion. Weitere geeignete Verbindungen sind amphiphile Moleküle mit den genannten funktionellen Gruppen, die sich in die Lipidschicht einlagern, ohne diese dabei zu zerstören. Dazu gehören unter anderem: Alkylamine, Alkylthiole und Fettsäuren sowie die aktivierten Ester solcher Fettsäuren. Weitere geeignete Verbindungen sind Sterolderivate, wie z. B. Cholsäuren, Deoxycholsäuren, Thiocholesterol und ähnliche Verbindungen.

Die Oberfläche von Liposomen kann durch Einführung reaktiver Gruppen chemisch modifiziert werden. Dazu gehören die Aktivester von membranständigen Carboxylfunktionen, etwa deren N-Hydroxysuccinimidster. Dazu gehören weiterhin Aldehydfunktionen, die sich etwa durch Behandlung von membranständigen Aminfunktionen mit Glutaraldehyd oder durch Oxidation glykosylierte Lipide erzeugen lassen. Dazu gehören weiterhin Thiolfunktionen, die sich etwa durch Reaktion membranständiger Aminofunktionen mit 2-Iminothiolan erzeugen lassen. Dazu gehören weiterhin membranständige 2-Pyridyldithionate oder Maleimide oder Haloacetyle, die sich durch geeignete heterobifunktionelle Reagenien erzeugen lassen.
Die Erzeugung solcher reaktiver Gruppen wird vorteilhaft nach der Herstellung der Liposomen durchgeführt, um diese Gruppen auf die Außenseite der Liposomen zu beschränken. Werden solche reaktiven Gruppen an membranbildenden oder membranständigen Komponenten bereits zur Bildung der Liposomen verwendet, so ist eine im

Einzelfall nachteilige Reaktion mit eingeschlossenen Cargomolekülen möglich.

Homo- oder heterofunktionelle Verknüpfungen zwischen Amino-, Thiol-, Hydrazo-, Aldehyd-, Carboxyl,-, Aktivester-, Hydroxyl- oder Azidwasserstoffgruppen werden vorteilhaft unter Zuhilfenahme von Hilfsstoffen ausgeführt. Dazu sind die in der Proteinchemie gebräuchlichen bifunktionellen Quervernetzer geeignet, wie z. B. Isothiocyanat, Isocyanate, Acylazide, N-Hydroxysuccinimidester, Sulfonylchide, Aldehyde, Epoxide, Carbonate, Imidoester, Carbodiimide, Anhydride, Haloacetyle, Alkylhalide, Maleimide, Aziridine, Pyridiyldisulfide, Diazoalkane, Diazoacetyle, Carboyldiimidazole, N-Hydroxysuccinimidylchloroformiate oder Verbindungen, die diese funktionellen Gruppen in geeigneten Kombinationen enthalten. Eine gebräuchiche Variante der Kopplung zwischen Amino- und Carboxylgruppen beinhaltet die Derivatisierung des Carboxyls in einem reaktiven Ester, etwa unter Verwendung von N-Hydroxysuccinimid. Diese Derivatiierung kann am Polymer oder am Liposom erfolgen.

Für eine nicht kovalente Kopplung insbesondere von Proteinen sind Chelatkomplexe geeignet. Proteine für eine solche Kopplung können solche sein, die natürlicherweise Chelatkomplexe bilden, etwa die DNA bindenden Zn-Fingerproteine. Mit Hife rekombinanter DNA-Techniken lassen sich aber auch chelatisierende Sequenzen in Proteine einfügen. Ein allgemein bekanntes Beispiel sind die als His-tag bekannten Hexahistidinextensionen an den N- oder C-Termini von Proteinen.

Solche Proteine können in Anwesenheit von Ionen der Übergangsmetalle an chelatisierende Lipidschichten binden. Diese Lipidschichten können durch den Einbau solcher amphiphiler Verbindungen erzeugt werden, die in ihrem polaren Teil die Trinitriloessigsäure oder die Diiminoessigsäure enthalten.

Ist das Polymer P1 ein Polymer mit hinreichender Affinität zur Lipidschicht, so ist kein distinkter Kopplungsschritt notwendig.
Zu den geeigneten Polymeren P1 gehören daher integrale und periphere Membranproteine, aber auch solche Polymere, deren Affinität zur Membran durch eine nachträgliche Modifizierung erhöht wurde. Methoden zu einer solchen Modifizierung beinhalten die Dotierung von Polymeren mit funktionalisierten Alkylresten, etwa die Behandlung mit N-Hydroxysuccinimidestern langkettier Fettsäuren oder auch die kovalente Kopplung von Phopholipiden. Eine solche Kopplung kann über vorhandene Amino-, Thiol- oder Carboxylgruppen unter Zuhilfenahme von homo- oder heterobifunktionellen Vernetzern erreicht werden, wobei das Phospholipid durch Detergenzien in Lösung gehalten wird. An glykosylierten Proteine lassen sich durch Oxidation Aldehydfunktionen erzeugen, die zur Kopplung an lipidständige Aminofunktionen genutzt werden können. Hierbei ist kein Hilfsstoff notwendig.
Analoge Modifizierungen sind auch an anderen natürlichen und synthetischen Polymeren möglich.

Synthetische Polymere können eine erhöhte Affinität zur Membran besitzen, wenn bei ihrer Herstellung amphiphile Komonomere zugemischt werden.
Proteine können auch durch molekularbiologische Verfahren in ihren Eigenschaften so verändert werden, daß sie zu integralen oder peripheren Membranproteinen werden.

Eine elektrostatische Aufkonzentrierung von P1 an der Lipidschicht ist vorteilhaft für die Ausführung des Kopplungsschritts. Dazu kann die Lipidschicht mit geeigneten ionischen Komponenten dotiert werden. Zu den geeigneten Komponenten gehören Alkylcarbonsäuren, Alkylsulfonsäuren, Alkylamine, Alkylammoniumsalze, Phosphorsäureester mit langkettigen Alkoholen, aber auch natürliche oder synthetische geladene Lipide, wie etwa Phosphatidylglycerol, Phosphatidylserin, geladene Derivate des Phosphatidylethanolamins oder Cholesteols, Phosphatidylinositol, Cardiolipin oder Sphingolipide.

Zur erfindungsgemäßen Ausbildung von Nanokapseln sollte eine hohe Beladung der Liposomen mit dem Polymer erreicht werden. Andererseits gilt es, die Bildung von Aggregaten möglichst zu unterdrücken. Wo möglich, sollten Lipidschicht und Polymer daher unterschiedliche funktionelle Gruppen besitzen. So können thiolhaltige Lipidschichten mit einer Vielzahl von thiolfreien Polymeren beschichtet werden, unter anderem auch mit Proteinen, die keine freie Thiolfunktion besitzen. Gerade bei der Verwendung von Proteinen als Polymer P1 läßt sich dies nicht immer bewerkstelligen. Unter diesen Umständen können vorteilhaft solche heterobifunktionellen Reagenzien als Hilfsstoffe eingesetzt werden, bei denen sich stabile Intermediate isolieren lassen, die dann eine gerichtete Reaktion ermöglichen.

Ein nach der Bindung eventuell vorhandener Überschuß an Polymer P1 kann durch geeignete Maßnahmen wie etwa Dialyse, Tangentialdialyse, Flotation, Gelfiltration oder Ultrafiltration entfernt werden.

In einem folgenden Schritt wird das Polymer P1 durch ein davon verschiedenes Polymer P2 kovalent vernetzt. Die dafür eingesetzten Hilfsstoffe und Verfahren entsprechen denen für die kovalente Fixierung von P1. Die Verwendung von Hilfsstoffen entfällt, wenn P1 und P2 von sich aus kovalente Verbindungen eingehen können. Das ist beispielsweise der Fall, wenn P1 oder P2 ein polyfunktionelles Aldehyd und der andere Partner ein polyfunktionelles Amin oder Hydrazin sind.

Sowohl P1 als auch P2 sind wasserlösliche Polymere, die eine größere Anzahl funktioneller Gruppen wie Amino-, Carboxyl-, Thiol, Hydrazo-, Hydroxyl-, Azidwasserstoff-, Aldehyd- oder Aktivestergruppen besitzen.

Dazu gehören insbesondere Polysaccharide wie Alginsäure, Chitosan, Pektin, Hyaluronsäure, Polymannuronsäure, Heparin, Gummi Arabicum, Karajagummi, Xanthangummi, Karragenan, Locus Bean Gum und die Salze dieser Verbindungen sowie carboxylierte, aminierte, thionylierte hydrazylierte oder oxidierte Dextrane, Stärken, Levane, Inuline oder Agarosen.
Dazu gehören auch das Polymerisationsprodukt des Glutaraldehyds und andere polyfunktionelle Aldehyde.
Dazu gehören weiterhin natürliche oder synthetische Proteine oder Peptide oder Homo- oder Heteropolymere aus Aminosäuren, die über mehrere freie Amino-, Carboxyl- oder Thiolgruppen verfügen.
Dazu gehören Polyacrylsäuren, Polyacrylamide, Polymethacrylsäure, Polymethacrylamide, Polyvinylpyrrolidone, Polyhydroxyethylacrylate, Polyhydroxymethylacrylate, Polyethylenimine und verzweigte Polyethylenglykole, die über mehrere freie Amino-, Carboxyl- oder Thiolgruppen verfügen. Diese funktionellen Gruppen können durch Copolymerisation oder durch nachträgliche Modifizierung eingefügt werden.

Vorteilhafte Varianten der Erzeugung solcher Copolymere sind in Hansen (Analytical Biochemistry 76:37 (1976)) oder in O'Connell und Brady (Analytical Biochemistry 76: 63 (1976)) beschrieben. Dabei wird Polyacrylamid in Gegenwart von spaltbaren Biacrylen polymerisiert. Hansen verwendet dazu N,N'-bis(Acryloyl)-Cystamin und spaltet das entstandene Gel reduktiv. Es entsteht ein Polythiol auf Acrylbasis, dass sich hervorragend zur Vernetzung im Sinne dieser Erfindung eignet. O'Conell und Brady setzen ein bifunktionelles Acrylamid mit zwei vicinalen Hydroxyfunktionen ein, die anschließend oxidativ gespalten werden. Es entsteht ein multivalenter Aldehyd, mit dem sich vernetze Hüllschichten aufbauen lassen.

Es gehören weiterhin dazu Mischformen der aufgeführten Verbindungen wie glykosylierte Proteine, posttranslational modifizierte Proteine, Proteinkomplexe mit anderen Naturstoffen, Kopolymere aus Zuckern und Acrylaten und verwandte Verbindungen, insofern als alle diese Verbindungen wasserlöslich sein müssen und nicht selbst micellare oder vesikuläre Strukturen bilden.

Es gehören weiterhin dazu modifizierte Polymere P1 oder P2, die zur Ausbildung von Chelatkomplexen befähigt sind oder die eine Affinität zu Lipidschichten haben.
Dazu gehören auf chemischem Wege erzeugte Derivate der bis hierher genannten Polymere. Dazu gehören auch solche synthetische Polymere, die ein amphiphiles oder ein chelatisierendes Comonomer enthalten. Dazu gehören auch gentechnisch veränderte Proteine mit chelatisierenden Eigenschaften.

Ein wichtiger Anwendungsfall besteht darin, daß eines der Polymere P1 oder P2 ein Protein ist.

Vorzugsvarianten sind solche, bei denen dieses Protein ein Albumin, ein Hämoglobin, ein Myoglobin, ein Antikörper, α2-Makroglobulin, Fibrinogen, Fibronectin, Collagen, Vitronectin, Protein A, Protein G, Avidin, Streptavidin, Concanavalin A, Wheat Germ Agglutinin oder ein Selektin ist.

Vorteilhaft ist es, wenn eines der Polymere P1 oder P2 eine fädige Struktur aufweist. Das ist bei vielen Kohlenhydraten der Fall oder bei Polymeren der Acrylsäure oder ihrer Derivate.

Das Polymer kann auch fluorescierende Eigenschaften haben oder diese durch Modifizierungen erhalten. Ein geeigneter Stoff mit solchen Eigenschaften ist das Green Fluorescent Protein. Andere Proteine oder Kohlenhydrate lassen sich mit fluorescierenden Stoffen modifizieren. Geeignete Methoden dazu sind dem Fachmann an sich bekannt und beinhalten die kovalente Bindung des aktivierten Fluorophors an entsprechende Gruppen des Polymers oder die Komplexbildung von fluorescierenden Metallionen mit chelatisierenden Gruppen des Polymers. Nanokapseln lassen sich auch nachträglich mit fluorescierenden Stoffen modifizieren.

Freie Nukleinsäuren gehören nicht zu den geeigneten Polymeren.

Da das Polymer P1 an der Oberfläche der Liposomen eine lokal weit höhere Konzentration als in freier Lösung aufweist, geht die Vernetzung vorzugsweise an der Oberfläche vonstatten. Reste von P1 in freier Lösung können mit dem Polymer P2 ebenfalls vernetzt werden und Partikel bilden. Freies P2 und freie P1-P2-Partikel lassen sich von den beschichteten Liposomen durch Dialyse, Tangentialdialyse, Flotation, Gelfiltration oder Ultrafiltration abtrennen.

Nach Beschichtung und Vernetzung erhält man Hohlkugeln, bei denen eine innere Lipidmembran mit einer äußeren polymeren Hülle umgeben ist. Diese Hülle verändert die Oberflächeneigenschaften der Liposomen und erhöht deren Stabilität.

In einer bevorzugten Variante der Erfindung werden Nanokapseln hergestellt, bei denen die Liposomen nach der Vernetzung aufgelöst wurden. Dies kann vorzugsweise durch Auswaschen mit einem Detergenz erfolgen.

Dabei kommt es auch zur Freisetzung von solchen Polymeren P1 oder P2, die lediglich an der Lipidschicht, nicht aber untereinander gebunden sind, sowie zum Zerfall nicht hinreichend vernetzter Strukturen. Die Nanokapseln lassen sich von diesen Zerfallsprodukten durch Sedimentation, Gelfiltration oder Ultrafiltration abtrennen. Geeignete Detergenzien sind alkylierte Zucker wie etwa Octylglucosid, Salze der Cholsäure und ihrer Derivate, Alkylsulfonsäuren oder Polyoxyethylensorbitole.

Hohlkugeln im Nanometerbereich im Sinne dieser Erfindung bestehen aus den beiden Polymeren P1 und P2. Die formgebenden Liposomen können erhalten bleiben oder entfernt werden. Die Größe der entstandenen Hohlkugeln wird durch die der eingangs verwendeten Liposomen bestimmt.

Die in der vorliegenden Erfindung beschriebenen Nanokapseln sind zum Einschluß von biologisch aktiven Verbindungen, beispielsweise von pharmazeutischen Wirkstoffen oder von Proteinen oder Nukleinsäuren, geeignet.

In diesem Fall werden Liposomen verwendet, welche die einzuschließenden Stoffe bereits enthalten. Methoden zur Herstellung solcher Liposomen sind dem Fachmann bekannt. Die einzuschließenden Verbindungen sind lediglich insofern eingeschränkt als sie die Integrität der Liposomen nicht nachteilig beeinflussen dürfen, wie etwa Detergenzien. Die eingeschlossenen Verbindungen verbleiben bei den weiteren Reaktionsschritten des Aufbringens von P1 und P2 in den Liposomen.

In die erfindungsgemäßen Nanokapseln können synthetisch chemische Verbindungen, Proteine, Peptide, Vitamine, Hormone, Kohlenhydrate oder Nukleinsäuren sowie Gemische derselben eingeschlossen werden, die beispielsweise als Antibiotika, Fungizide und antivirale Agenzien, Antikörper, Zytostatika und Immunsuppressiva, Analgetika, Anästhetika, Antidepressiva, Antidiabetika, Antihypertensika, Antikoagulatien, antiinflammatorische, angstlösende, sedative, antiarrhythmische, antiarthritische Wirkstoffe, Bronchodilatoren, hypoglykämische und hypolipidämische Wirkstoffe sowie Wirkstoffe zur Stimulierung der Erythropoese dienen können.

Die Permeabilität der Hüllschicht der Nanokapseln wird durch das Auswaschen der Liposomen wesentlich erhöht. Dieser Prozeß beinhaltet die Passage von Detergensmolekülen und Mischmicellen durch die äußere Hüllschicht. In gleicher Weise können Substrate und Produkte einer im Innern der Nanokapsel stattfindenden Reaktion ausgetauscht werden. Eine Anordnung zur Durchführung solcher Reaktionen besteht vorzugsweise aus Nanokapseln mit im Inneren befindlichen enzymatisch aktiven Stoffen mit hohem Molekulargewicht, deren Liposomen durch Detergenzien ausgewaschen wurden. Geeignete Stoffe für einen solchen Einschluß sind insbesondere Enzyme oder Ribozyme.
In einer anderen Variante dieser Ausgestaltung der Erfindung bleibt die Lipidschicht erhalten. In dieser Ausgestaltung können nur solche Stoffe ausgetauscht werden, die durch die Lipidschicht diffundieren.

In einer vorteilhaften Ausgestaltung der Erfindung werden solche Polymere zum Aufbau der Hüllschicht verwendet, die nicht nur strukturbildend, sondern auch aktivitätstragend sind. Solche Hüllen können zum Beispiel Bindungseigenschaften für andere Moleküle oder katalytische Eigenschaften besitzen. Unter den Proteinen finden sich solche Polymere mit strukturbildenden und aktivitätstragenden Eigenschaften.
In einer Variante dieser erfinderischen Ausgestaltung wird Hämoglobin zum Aufbau der Hüllstruktur benutzt. Die entstehenden Nanokapseln können als Blutersatz verwendet werden.

In einer andern Variante dieser Ausgestaltung wird die Hüllschicht unter Verwendung von solchen Proteinen hergestellt, die häufig vorkommende Merkmale anderer Proteine erkennen und binden können. Geeignete Proteine für diesen Zweck sind Lektine, biotinbindende oder antikörperbindende Proteine. Mit dieser Variante der Erfindung werden Nanokapseln erzeugt, die Glykosylierungen, antigene Epitope oder Biotingruppen auf Proteinen erkennen können und diese Proteine hochspezifisch binden. Solche Nanokapseln sind von Interesse für die biochemische Diagnostik. Nanokapseln mit einem solchen Aufbau können aber auch für eine zielgesteuerte Applikation von Arzneistoffen benutzt werden.
Zu den hochspezifischen Molekülen gehören daher insbesondere solche, die mit der Oberfläche von Zellen interagieren können. Komplementäre Paare in diesem Sinne sind Antikörper und membranständige Antigene, Lektine oder Selektine und membranständige Glykosylierungen, Hormone und deren Rezeptoren und andere mehr.
Vorteilhaft ist der modulare Aufbau der Strukturen, der zum einen die Erzeugung einer offenen Anzahl von Spezifitäten auf einigen wenigen Hüllschichten erlaubt, zum anderen einen sehr ökonomischen Einsatz der letztlich spezifitätsbestimmenden Komponenten gestattet.

Darüber hinaus kommen diese Komponenten nicht mit den zur Vernetzung verwendeten Chemikalien in Kontakt, die Gefahr einer Inaktivierung ist daher nicht gegeben. Die Valenz der erhaltenen Struktur, das heißt die Anzahl der oberflächlich gebundenen spezifischen Komponenten läßt sich leicht durch Titration verändern. Eine hohe Dichte dieser Komponenten ist gleichbedeutend mit einer hohen Avidität und ermöglicht stabile Interaktionen auch bei ungünstigen Bindungskonstanten der einzelnen Wechselwirkung, wie sie etwa zwischen MHC-Komplexen und T-Zell-Rezeptoren gegeben ist.

In einer weiteren vorteilhaften Ausgestaltung der Erfindung werden Nanokapseln nach ihrer Entstehung mit weiteren Stoffen modifiziert. Eine wichtige Variante dieser Ausgestaltung ist die Modifizierung der Oberfläche der Nanokapseln mit Polyethylenglykol. Eine solche Beschichtung führt zu Partikeln mit einer verbesserten Verträglichkeit bei pharmazeutischen Anwendungen.

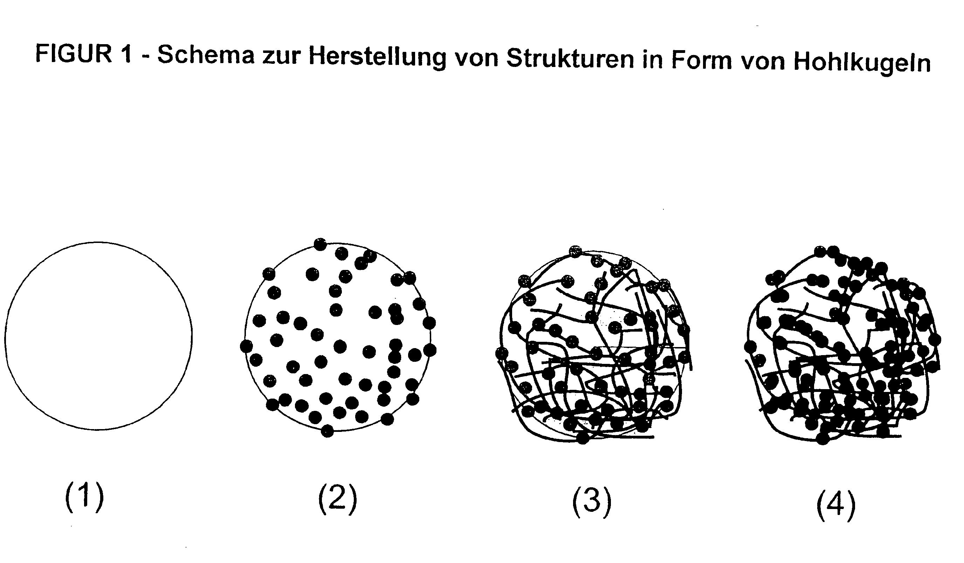

Figur 1 zeigt ein Schema zur Herstellung der erfindungsgemäßen Nanokapseln:
Liposomen (1) werden zunächst mit dem Polymer P1 beschichtet (2). Anschließend wird diese Schicht durch eine anderes Polymer P2 vernetzt (3). Die formgebenden Liposomen können durch Detergenzien entfernt werden (4).

Die Verwendung der erfindungsgemäßen Nanokapseln erfolgt vor allem als Container und Transporter für biologisch wirksame Stoffe.

In einer bevorzugten Verwendung kommen insbesondere solche Stoffe mit enzymatischer Aktivität zum Einsatz, deren Substrate und Produkte durch die Hüllschicht ausgetauscht werden können.

Nanokapseln im Sinne der vorliegenden Erfindung besitzen eine diffusionsoffene Struktur, die den Austausch von Molekülen mit signifikanter Größe, etwa beim Herauslösen der Lipidschicht, zuläßt. Große Moleküle wie etwa Enzyme werden jedoch von der Hüllschicht zurückgehalten. In weiteren erfindungsgemäßen Verwendungen der Nanokapseln sind diese mit solchen Enzymen gefüllt, die Reaktionen katalysieren, deren Substrate und Produkte die Hüllschicht passieren können. Diese Art der Verpackung eines biologischen Makromoleküls in Nanokapseln hat gegenüber dem Stand der Technik den Vorteil extrem geringer Diffusionswege und einer damit verbundenen Erhöhung der spezifischen Aktivität des eingeschlossenen Enzyms. Darüber hinaus kann die Einwirkung von vernetzenden Agenzien, wie sie bei der chemischen Fixierung auftritt, vermieden werden.

In einer weiteren erfindungsgemäßen Verwendung werden signalgebende Systeme, wie etwa Meerrettich-Peroxidase oder alkalische Phosphatase oder fluorescensmarkierte Makromoleküle, in solche Nanokapseln eingeschlossen, die spezifische Bindungseigenschaften gegenüber anderen Stoffen aufweisen. Solche Systeme sind zur Detektion dieser anderen Stoffe geeignet, insbesondere in der medizinischen oder biochemischen Diagnostik. Vorteilhaft gegenüber den Liposomen ist die Tatsache, daß Nanokapseln stabil gegenüber Detergenzien sind, insbesondere auch gegenüber solchen Detergenzien, die zur Unterdrückung unspezifischer Bindungen in solchen Verfahren eingesetzt werden, wie etwa Tween 20 oder Triton X-100.

In einer Variante dieser erfindungsgemäßen Verwendung sind die Nanokapseln selbst Träger des signalgebenden Systems. Vorteilhaft werden Nanokapseln präpariert, deren Polymere fluorescierende Eigenschaften besitzen. Dabei werden fluorescierende Derivate von P1 und/oder P2 zum Aufbau der Nanokapseln verwendet oder die Nanokapseln nach ihrer Herstellung mit fluorescierenden Stoffen kovalent verbunden.

Die hier beschriebenen Strukturen sind als Träger für pharmazeutische Wirkstoffe im Sinne des drug delivery, im Sinne eines Transfervektors, im Sinne eines Depotsystems oder bei einer Enzymersatztherapie geeignet. Gegenstand der Erfindung ist deshalb auch die Verwendung der erfindungsgemäß hergestellten Nanokapseln zur Herstellung von pharmzeutischen Zubereitungen, die der Applikation von Wirkstoffen - wie oben beschrieben - dienen. Bei einer anderen Verwendung in Detektionssystemen, also in der biochemischen Diagnostik, ist die Stabilität der Struktur gegenüber Detergenzien von wesentlichem Vorteil, da solche Stoffe üblicherweise zur Unterdrückung unspezifischer Interaktionen eingesetzt werden.

In einer erfindungsgemäßen Verwendung der Nanokapseln sind diese so beschaffen, daß sie spezifisch an Zielzellen von Säugetieren binden. Nanokapseln im Sinne dieser Verwendung besitzen eine oder mehrere Klassen von Liganden auf ihrer Oberfläche, deren komplementäre Bindungspartner sich auf der Oberfläche der Zielzellen befinden. Nanokapseln mit solchen Eigenschaften sind Träger für Therapeutika, die diese an eine definierten Wirkort dirigieren. Die innere Lipidschicht der Hohlkugeln kann bei dieser Verwendung erhalten bleiben, wenn es dem Einschluß der zu transportierenden Substanz dienlich ist.

In einer Variante dieser erfindungsgemäßen Verwendung enthalten die Nanokapseln Stoffe, gegen die eine Immunantwort ausgelöst werden soll.

In einer weiteren vorteilhaften Variante dieser Ausgestaltung der Erfindung werden die Nanokapseln zum Transfer von Wirkstoffen in das Cytosol von Säugetierzellen benutzt. Diese Nanokapseln sind so beschaffen, dass sie von Säugetierzellen endozytiert werden. Nanokapseln für diese Ausgestaltung der Erfindung bestehen aus einer Hüllschicht, die von den Hydrolasen des Endosoms abgebaut werden kann. Sie werden darüberhinaus aus solchen Liposomen hergestellt, deren Membran mit der des endozytotischen Vesikels fusionieren kann. Vorteilhaft bei dieser Ausgestaltung der erfinderischen Lehre ist die Tatsache, dass eine solche Fusion nicht zu einer Freisetzung lytischer endosomaler Aktivitäten in das Zellinnere führen kann. Nanokapseln für diesen Verwendungszweck können mit unterschiedlichen Wirkstoffen beladen werden. Der beschriebene Transportweg ist jedoch von besonderem Vorteil beim Transport nicht membrangängiger biologischer Makromoleküle, wie etwa Proteine, Peptide, Antikörper, Enzyme, Oligonukleotide, DNA, RNA, Hormone, aber auch von Antibiotika, Fungiziden und antivirale Agenzien sowie von Zytostatika.

Nanokapseln gemäß der hier vorliegenden Erfindung sind hydrophile, permeable und detergensstabile Strukturen aus vernetzten Polymeren, die sich aufgrund der Vielzahl von verwendbaren Komponenten für eine große Zahl von Anwendungen spezifizieren lassen. Die vorliegende Erfindung erweitert erheblich das Spektrum solcher Stoffe, die sich als Trägermaterialien im Sinne eines drug targeting, eines Transfervektors, einer Depotform oder für eine Enzymersatztherapie verwenden lassen. Dabei können die verwendeten Komponenten sowohl strukturbildend als auch aktivitätstragend sein. Die beschriebenen Hohlkugeln lassen sich aus Stoffen mit einer antigenen Wirkung herstellen oder aus solchen, die keine Immunantwort hervorrufen.

Benutzt man die hier beschriebene Struktur zum Einschluß von Enzymen, so ist durch die diffusionsoffene Architektur eine hohe Verfügbarkeit der eingeschlossenen Aktivität gewährleistet. Bei der gewählten Größe im Mikrometer- und Submikrometerbereich sind die Diffusionswege zudem extrem kurz. Während der Präparation der Hüllen sind die eingeschlossenen Stoffe vor der Aktion chemischer Quervernetzer geschützt, es kann damit nicht zu einer Inaktivierung durch diese Chemikalien kommen. Der hier formulierte Einschluß von Makromolekülen ist daher der denkbar schonendste.

### Beispiele

Verwendete Abkürzungen
- PC: Phosphatidylcholin
- PS: Phosphatidylserin
- HEPES: N-[2-Hydroxyethylpiperazin-N'-(2-ethansulfonsäure)]
- MES: 2-(N-Morpholino)-ethansulfonsäure
- Sulfo-SMCC: Sulfosuccinimidyl-4-[N-maleimidomethyl]-cyclohexan-1-carboxylat
- BSA: Rinderserumalbumin
- EDC: 1-Ethyl-3-(3-dimethylaminopropyl)-carbodiimid
- CHAPS: 3-[(Cholamidopropyl)-dimethylammonio]-2-hydroxypropansulfonsäure
- DeoxyBigCHAP: N,N'-bis(3-Gluconamidopropyl)-deoxycholamid
- EDTA: Ethylendiamintetraessigsäure

### Beispiel 1 Herstellung von BSA-Alginat-Nanokapseln

### Herstellung der Liposomen

Die als Matrix verwendeten Liposomen werden durch Dialyse eines wässrigen Gemisches aus PC (47,5 mol%), PS (2,5 mol%) und Natriumdeoxycholat (50 mol%) hergestellt. Das Gemisch wird gegen 150mM Natriumchlorid in Wasser dialysiert.

### Beschichtung mit P1

Zur Beschichtung mit BSA stellt man folgende Endkonzentrationen ein: Liposomen 4mg/ml, BSA 10mg/ml, EDC 10mg/ml und MES 50mM pH5,1. Die Fixierung des BSA auf der Oberfläche der Liposomen erfolgt für mindestens eine Stunde bei 37°C, anschließend beendet man die Reaktion durch Zugabe von 200mM Kaliumacetat. Der Überschuß des eingesetzten BSA und EDC wird durch Flotation abgetrennt.

### Beschichtung mit P2

Zur Vernetzung der P1-Schicht werden den beschichteten Liposomen 200µg/ml Natriumalginat und 50mM MES-Puffer pH5.1 zugesetzt. Die Vernetzung wird durch Zugabe von 10mg/ml EDC gestartet und für zwei Stunden bei 37°C durchgeführt. Die Reaktion wird anschließend wie oben durch Zugabe von 200mM Kaliumacetat gestoppt.

### Auswaschen der Liposomen

Zur Entfernung der Liposomen wurden die beschichteten Liposomen mit 1% CHAPS behandelt. Die so erhaltenen detergensstabilen Strukturen entsprechen in ihrer Größe dem Ausgangsmaterial. Die Effizienz der Kapselbildung wird durch Messung der Lichtstreuung vor und nach der Detergensgabe bestimmt und liegt zwischen 30 und 60%.

### Beispiel 2

### Herstellung von Kapseln aus einem Lektin und Alginat

### Herstellung der Liposomen

820mg PC werden in 1ml Ethanol gelöst; 42mg PS und 490mg Natriumdeoxycholat werden in 2.5ml Wasser gelöst. Beide Lösungen werden vereinigt und mit 150mM NaCl auf 40ml aufgefüllt. Liposomen werden daraus durch Gelfiltration an Sephadex® G-25 in 150mM NaCl hergestellt. Die gewonnenen Liposomen werden durch Flotation in einer Ultrazentrifuge aufkonzentriert, kurz mit Ultraschall behandelt und durch ein Filter der Porenweite 0,22µm steril filtriert.

### Beschichtung mit P1

5ml der Liposomen werden mit 1ml MES-Puffer (500mM pH5) und 0,3ml NaCl-Lösung (5M) versetzt, anschließend werden 35mg Concanavalin A (SIGMA, type VI) und 75mg EDC zugesetzt und die Mischung für 3h bei 37°C inkubiert. Die Reaktion wird durch Zugabe von 2ml HEPES (1M pH8) und 0,2ml Kaliumacetatlösung (5M) gestoppt. Die mit P1 beschichteten Liposomen werden durch Flotation in der Ultrazentrifuge isoliert und anschließend in 5ml MES-Puffer (100mM pH5) aufgenommen.

### Vernetzung mit P2

2,5ml der mit Concanavalin A beschichteten Liposomen werden 0,1ml Natriumalginat (10mg/ml) und 50mg EDC gemischt. Die Lösung wird mit 100mM MES-Puffer pH5 und 200mM NaCl auf 4ml aufgefüllt und über Nacht inkubiert. Zum Abschluß der Reaktion werden 1ml HEPES (1M pH8) und 0,2ml Kaliumacetat (5M) sowie je 5µl CaC12 und MnCl2 (je 1M) zugesetzt.

Auswaschen der Liposomen und Isolierung der Nanokapseln Der Ansatz wie oben wird mit 2,5mg DeoxyBigCHAP behandelt. Die Isolierung der Hüllstrukturen gelingt durch Sedimentation in der Ultrazentrifuge unter Nutzung eines Sucrosegradienten. Nanokapseln sedimentieren durch eine 0,5M Sucroseschicht und werden an der Grenzfläche zu einer 2M Sucroselösung zurückgehalten. Die so erhaltenen Proben enthalten kein nachweisbares Lipid und 1mg/ml Protein. Das verwendete Concanavalin A ist nach dem Einbau in die Hüllschicht noch bindungsfähig und kann glykosylierte Proteine binden.

### Beispiel 3

### Bindung glykosylierter Proteine an Nanokapseln aus Concanavalin A - Alginat

Sec-Komplex aus Saccharomyces cerevisiae wird wie in Panzner et al., Cell 1995, May 19; 81(4):561-70 beschrieben aufgereinigt. 2...20µl der Nanosphären aus Beispiel 2 und 20µl des Sec-Komplexes werden mit 80µl Puffer (50mM HEPES pH 7.5, 0.5%DeoxyBigCHAP) vereinigt und für 12h bei 4°C inkubiert. Die enthaltenen Nanosphären werden sedimentiert (Rotor Beckman 100.3, 75.000rpm für 30min) und die Verteilung des Sec-Komplexes wird mittels SDS-PAGE analysiert. 5µl der präparierten Nanospheren führen zu einer Bindung von mehr als der Hälfte des angebotenen Sec-Komplexes.

### Beispiel 4

### Einschluß von Peroxidase in Nanokapseln

Liposomen werden wie unter 2. durch Gelfiltration hergestellt, wobei der Ausgangslösung 1mg/ml Meerrettich-Peroxidase (POD) zugesetzt werden. Nicht eingeschlossene POD wird bei der Flotation abgetrennt. 1.3% der anfänglichen Enzymmenge und 25% des eingesetzten Lipids finden sich in der flotierten Phase. Die Beschichtung wird wie unter 2. mit ConcanavalinA und Alginat vorgenommen.
100µl der liposomalen Nanokapseln und 100µl unbeschichtete Liposomen wurden zur Analyse des Einschlusses verwendet. Dazu wurden beide Proben mit je 100µl Detergenslösung (2% DeoxyBIGChap, 100mM HEPES pH7.5 und 150mM Kaliumacetat) gemischt und in einem Ultrazentrifugationröhrchen (0.8ml für Beckman SW55) mit 350µl einer Sucroselösung mittlerer Dichte (0.8M Sucrose, 50mM HEPES pH7.5, 150mM Kaliumacetat und 0.2% DeoxyBIGChap) und 100µl einer Sucroselösung hoher Dichte (2M Sucrose, 50mM HEPES pH7.5, 150mM Kaliumacetat und 0.2% DeoxyBIGChap) unterschichtet und für 1h bei 55.000rpm zentrifugiert.
Von der Phasengrenze zwischen 2M und 0.8M Sucrose wurden die Nanokapseln gesammelt, freigesetzte Proteine und zerfallene Hüllschichten wurden aus der obersten, Probenauftragsschicht entnommen. Die Verteilung von Protein, Lipid und POD ist für unbeschichtete Liposomen und Nanokapseln in der untenstehenden Tabelle wiedergegeben.
Nur nach Beschichtung der Liposomen mit ConcanavalinA und Alginat findet sich ein signifikanter Anteil (etwa 25%) der POD gemeinsam mit etwa 15% des Proteins in der sedimentierten Phase.

| | Liposomen, obere Phase | Liposomen, untere Grenzschicht | Nanokapseln, obere Phase | Nanokapseln, untere Grenzschicht |
|---|---|---|---|---|
| Lipidverteilung | 100% | 0% | 100% | 0% |
| Proteinverteilung | 97% | 3% | 87.5% | 12.5% |
| POD-Verteilung | 100% | 0% | 73% | 27% |

### Beispiel 5

### Herstellung von BSA-Alginat-Nanokapseln unter Verwendung thiolhaltiger Liposomen

### Herstellung der Liposomen

400mg PC und 7.5mg Octadecylmercaptan werden in 1ml Ethanol gelöst und anschließend unter Rühren in 40ml Puffer (10mM HEPES, 150mM NaCl, 5mM EDTA, pH7.5) gegeben. Die erhaltene liposomale Suspension wird bei 800bar mit einem Hochdruckhomgenisator behandelt und anschließend durch ein 0,45µm-Filter gedrückt.

### Beschichtung mit P1

Liposomen wie oben werden mit dem angegebenen Puffer auf 5mg/ml Lipid verdünnt. Anschließend werden BSA (2mg/ml) und sulfo-SMCC (2mM) zugesetzt. Die Mischung wird über Nacht bei Raumtemperatur inkubiert.

### Vernetzung mit P2

0.9ml der beschriebenen Reaktionsmischung werden mit folgenden Lösungen vermischt:
0.1ml MES-Puffer, 500mM pH5
0.08ml NaCl-Lösung, 5M
0.1ml Natriumalginat, 4mg/ml
0.1ml EDC, 100mg/ml

Die Vernetzung wird für zwei Stunden bei Raumtemperatur durchgeführt.

### Auflösung der inneren Liposomen

Die Auflösung der inneren Liposomen gelingt wie in den vorherigen Beispielen ausgeführt durch Zugabe von Detergenzien. Anhand der Änderung in der Intensität des Streulichts vor und nach der Zugabe des Detergens kann auf die Ausbildung eigenstabiler Hüllen geschlossen werden. Vorzugsweise wird 1%Natriumcholat zur Auflösung der Liposomen eingesetzt.

### Beispiel 6

### Verwendung ionisch geladener Liposomen zur Herstellung der Nanokapseln

### Herstellung der Liposomen

400mg PC, 7.5mg Octadecylmercaptan und 9.7mg Cetyltrimethylammoniumbromid werden in 1ml Ethanol gelöst und anschließend unter Rühren in 40ml Puffer (10mM HEPES, 150mM NaCl, 5mM EDTA, pH7.5) gegeben. Die erhaltene liposomale Suspension wird bei 800bar mit einem Hochdruckhomgenisator behandelt und dann durch ein 0,45µm-Filter gedrückt.

Beschichtung und Vernetzung und Auflösung der Liposomen können wie in Beispiel 5 ausgeführt werden. Durch die Aufkonzentrierung des (negativ geladenen) BSA an den positiv geladenen Liposomen wird eine schnellere Reaktion möglich, die Reaktionszeit kann auf zwei Stunden gesenkt werden.

### Beispiel 7

### Nanokapseln aus Hämoglobin und Alginat

### Herstellung der Liposomen

400mg PC und 7.5mg Octadecylmercaptan werden in 1ml Ethanol gelöst und anschließend unter Rühren in 40ml Puffer (10mM HEPES, 150mM NaCl, 5mM EDTA, pH7.5) gegeben. Die erhaltene liposomale Suspension wird bei 800bar mit einem Hochdruckhomgenisator behandelt und anschließend durch ein 0,45µm-Filter gedrückt.

### Beschichtung mit P1

Liposomen wie oben werden mit dem angegebenen Puffer auf 5mg/ml Lipid verdünnt. Anschließend werden Hämoglobin (2mg/ml) und sulfo-SMCC (2mM) zugesetzt. Die Mischung wird über Nacht bei Raumtemperatur inkubiert.

### Vernetzung mit P2

0.9ml der beschriebenen Reaktionsmischung werden mit folgenden Lösungen vermischt:
0.1ml MES-Puffer, 500mM pH5
0.08ml NaCl-Lösung, 5M
0.1ml Natriumalginat, 4mg/ml
0.1ml EDC, 100mg/ml

Die Vernetzung wird für zwei Stunden bei Raumtemperatur durchgeführt.

Die eigenständige Stabilität der entstandenen Hüllschicht kann wie im Beispiel 5 durch Zugabe eines Detergens und Messung der Lichtstreuung erfolgen.

### Beispiel 8

### Nanokapseln aus Hämoglobin und Chitosan

Liposomen werden wie in Beispiel 7 hergestellt und mit Hämoglobin beschichtet.

Nach Abschluß der Beschichtung mit Hämoglobin werden zu 0.9ml der beschriebenen Reaktionsmischung die folgenden Lösungen gegeben:
0.1ml MES-Puffer, 500mM pH5
0.08ml NaCl-Lösung, 5M
0.08ml Chitosan aus Krabben, 85% deacyliert, 5mg/ml
0.1ml EDC, 100mg/ml

Die Vernetzung wird für zwei Stunden bei Raumtemperatur durchgeführt.

Die eigenständige Stabilität der entstandenen Hüllschicht kann wie im Beispiel 5 durch Zugabe eines Detergens und Messung der Lichtstreuung erfolgen.

### Beispiel 9

### Nanokapseln aus anderen Proteinen und Chitosan

Die Vorschrift aus Beispiel 8 ist unter den gleichen Bedingungen mit Concanavalin A oder Collagen oder Albumin durchführbar.

### Beispiel 10

### Nanokapseln unter Verwendung eines Acrylats

Liposomen werden wie in Beispiel 5 hergestellt und mit BSA beschichtet.

### Herstellung eines thiolreaktiven Acrylats

Acrylate mit freien Thiolfunktionen lassen sich durch reduktive Zersetzung von disulfidvernetzten Polyacrylamidgelen erzeugen. Eine Arbeitsvorschrift zur Herstellung und Zersetzung solcher Gele ist bei Hansen (Analytical Biochemistry 76: 37-44 (1976)) gegeben. Nach dieser Vorschrift werden thiolhaltige Polyacrylamide mit einem Substitutionsgrad von mindestens 5% hergestellt.

### Vernetzung mit thiolreaktiven Acrylaten

Thiolreaktive Acrylate wie oben werden zu Liposomen gegeben, die wie in Beispiel 5 oder 7 oder 9 mit Proteinen beschichtet wurden. Die Endkonzentration des Polymers beträgt dabei 400µg/ml. Die Mischung wird für einige Stunden bei Raumtemperatur inkubiert, anschließend kann die eigenständige Stabilität der Hüllschicht durch Auswaschen der Liposomen wie in Beispiel 5 nachgewiesen werden.

### Beispiel 11

### Nanokapseln ohne Verwendung von Proteinen

Liposomen werden wie in Beispiel 5 hergestellt.

### Beschichtung mit P1

Liposomen werden mit dem in Beispiel 5 verwendeten Puffer auf 5mg/ml Lipid verdünnt. Anschließend werden Chitosan (0,25mg/ml) und sulfo-SMCC (2mM) zugesetzt. Die Mischung wird über Nacht bei Raumtemperatur inkubiert.

### Vernetzung mit P2

Zur Reaktionsmischung werden 400µg/ml des thiolhaltigen Polyacrylamids aus Beispiel 10 gegeben. Die Mischung wird für einige Stunden bei Raumtemperatur inkubiert, anschließend kann die eigenständige Stabilität der Hüllschicht durch Auswaschen der Liposomen wie in Beispiel 5 nachgewiesen werden.

Beschichtung mit einem Protein

### Beispiel 12

### Nanokapseln unter Verwendung membranständiger Proteine

### Modifizierung von BSA

50mg BSA werden in 2,5 ml Puffer (20mM Natriumphosphat, 150 mM NaCl, 40mM Natriumdeoxycholat, pH 7.5) gelöst, anschließend werden 1,25mg Palmitinsäure-N-Hydroxysuccinimidester zugesetzt. Die Mischung wird für 2 Stunden bei Raumtemperatur inkubiert, anschließend werden nicht gebundener Palmitinsäure-N-Hydroxysuccinimidester und seine Hydrolyseprodukte durch Gelfiltration an Sephadex G25 abgetrennt. Hierbei wird ein Puffer wie oben verwendet, der aber nur 4mM Natriumdeoxycholat enthält.

### Herstellung der Liposomen

400mg PC werden in 1ml Ethanol gelöst und rasch in 40ml Puffer (20mM Natriumphosphat, 150 mM NaCl, pH 7,5) verdünnt. Die erhaltene liposomale Suspension wird bei 800bar mit einem Hochdruckhomgenisator behandelt und anschließend durch ein 0,45µm-Filter gedrückt.

### Beschichtung mit modifiziertem BSA

Die Lipsomen werden mit dem modifizierten Protein gemischt und mit Puffer (20mM Natriumphosphat, 150mM NaCl, pH7.5) auf 50ml aufgefüllt. Dann wird soviel Natriumdeoxycholat zugesetzt, dass die Endkonzentration 10mM beträgt. Die Lösung wird unter leichter Bewegung für zwei Stunden bei Raumtemperatur inkubiert, anschließend wird das Detergens durch Dialyse gegen 20mM Phosphat, 150mM NaCl, pH7.5 entfernt.

### Vernetzung mit Alginat

Zur Vernetzung der BSA-Schicht werden den beschichteten Liposomen 200µg/ml Natriumalginat und 50mM MES-Puffer pH5.1 zugesetzt. Die Vernetzung wird durch Zugabe von 10mg/ml EDC gestartet und für zwei Stunden bei 37°C durchgeführt. Die Reaktion wird anschließend durch Zugabe von 200mM Kaliumacetat gestoppt.
Die eigenständige Stabilität der Hüllschciht kann durch Auflösung der inneren Lipidschicht mit einem Detergens und Vergleich der Intensität des Streulichts bestimmt werden.

### Beispiel 13

### PEG-modifizierte Nanokapseln

Nanokapseln werden wie im Beispiel 10 unter Verwendung eines thiolhaltigen Acrylats hergestellt. Nicht eingebaute Polymere werden durch Flotation entfernt, der verwendete Puffer enthält 10mM HEPES, 150mM NaCl, 5mM EDTA, pH7.5. Zu den flotierten Nanokapseln wird soviel Puffer gegeben, dass deren Lipidkonzentration 5mg/ml beträgt. Zu dieser Lösung werden 0,5mg/ml α-Methoxy-ω-Maleimido-Polyethylenglykol gegeben und für zwei Stunden bei Raumtemperatur inkubiert.

### Beipsiel 14

### Fluorescierende Nanokapseln

### Herstellung der Liposomen

Alle Handlungen sind in abgedunkelten Räumen vorzunehmen. 400mg PC und 7.5mg Octadecylmercaptan werden in 1ml Ethanol gelöst und anschließend unter Rühren in 40ml Puffer (10mM HEPES, 150mM NaCl, 5mM EDTA, 20mM Calcein, pH7.5) gegeben. Die erhaltene liposomale Suspension wird bei 800bar mit einem Hochdruckhomgenisator behandelt und anschließend durch ein 0,45µm-Filter gedrückt. Nicht in die Liposomen eingeschlossenes Calcein wird durch Gelfiltration an Sephadex G25 abgetrennt. Dazu wird ein Puffer verwendet, der 10mM HEPES, 150mM NaCl, 5mM EDTA, pH7.5 enthält.

Die Beschichtung der Liposomen kann wie in Beispiel 5 ausgeführt werden. Calcein wurde in diesem Beispiel in einer Konzentration eingeschlossen, die zur Selbstquenchung des ausgesendeten Fluorescenzlichts führt. Dieser Quencheffekt wird bei einem Austritt des Calceins in das umgebende Medium aufgehoben. Solche Nanokapseln können zur Bestimmung von Stabilitäten in verschiedenen Medien, insbesondere in biologischen Systemen wie Mageninhalt, Darminhalt, Serum, Lymphe verwendet werden.

## Patentansprüche

1. Verfahren zur Herstellung von Nanokapseln mit einem Durchmesser von 50 nm bis 10 *µ*m,
**dadurch gekennzeichnet, dass**
Liposomen hergestellt werden, diese mit einem Polymer P1 beschichtet werden, indem das Polymer P1 in wäßriger Lösung an die Liposomenoberfläche kovalent gebunden wird und dann das aufgebrachte Polymer P1 mit einem von P1 verschiedenen Polymer P2 in wäßriger Lösung kovalent vernetzt wird und gegebenenfalls noch weitere Polymerschichten durch Vernetzung aufgebracht werden.

2. Verfahren nach Anspruch 1,
**dadurch gekennzeichnet, dass**
die Liposomen nach Vernetzung der Polymere aufgelöst werden, vorzugsweise durch Auswaschen mit einem Detergenz.

3. Verfahren nach Anspruch 1 oder 2,
**dadurch gekennzeichnet, dass**
von Liposomen ausgegangen wird, die biologisch aktive Verbindungen oder Verbindungen eines Detektionssystems tragen, welche bei der Durchführung des Verfahrens in den Nanokapseln verbleiben.

4. Verfahren nach einem der Ansprüche 1 bis 3,
**dadurch gekennzeichnet, dass**
als wasserlösliche Polymere P1 und P2 solche eingesetzt werden, die als funktionelle Gruppen Amino-, Carboxyl-, Thiol-, Hydrazo-, Hydroxy-, Azidwasserstoff-, Aldehyd- und/oder Aktivestergruppen oder Kombinationen dieser Gruppen aufweisen und nicht selbst micellare oder vesikuläre Strukturen bilden.

5. Verfahren nach einem der Ansprüche 1 bis 4,
**dadurch gekennzeichnet, dass**
das Polymer P1 mit den Liposomen oder das Polymer P1 mit dem Polymer P2 durch Hilfsstoffe vernetzt wird.

6. Verfahren nach Anspruch 5,
**dadurch gekennzeichnet, dass**
als Hilfsstoffe Isothiocyanat, Isocyanate, Acylazide, N-Hydroxysuccinimidester, Sulfonylchide, Aldehyde, Epoxide, Carbonate, Imidoester, Carbodiimide, Anhydride, Haloacetyle, Alkylhalide, Maleimide, Aziridine, Pyridiyldisulfide, Diazoalkane, Diazoacetyle, Carboyldiimidazole, N-Hydroxysuccinimidylchloroformiate oder Verbindungen, die diese funktionellen Gruppen in geeigneten Kombinationen enthalten, eingesetzt werden.

7. Verfahren nach einem der Ansprüche 1 bis 3,
**dadurch gekennzeichnet, dass**
die wasserlöslichen Polymere P1 oder P2 chelatisierende oder chelatbindende Eigenschaften aufweisen.

8. Verfahren nach einem der Ansprüche 1 bis 6,
**dadurch gekennzeichnet, dass**
die Polymere P1 und/oder P2 Proteine sind.

9. Verfahren nach einem der Ansprüche 1 bis 6,
**dadurch gekennzeichnet, dass**
die Proteine ausgewählt sind aus der Gruppe umfassend Albumin, Collagen, Hämoglobin und/oder Fibrinogen.

10. Verfahren nach einem der Ansprüche 1, bis 6,
**dadurch gekennzeichnet, dass**
die Polymere P1 und/oder P2 Kohlenhydrate sind.

11. Verfahren nach einem der Ansprüche 1 bis 6,
**dadurch gekennzeichnet, dass**
die Kohlenhydrate ausgewählt sind aus der Gruppe umfassend Alginat, Heparin, Pektin, Chitosan und/oder Hyaluronsäure.

12. Verfahren nach einem der Ansprüche 1 bis 6,
**dadurch gekennzeichnet, dass**
die wasserlöslichen Polymere P1 und/oder P2 synthetische Polmyere sind.

13. Verfahren nach einem der Ansprüche 1 bis 6,
**dadurch gekennzeichnet, dass**
die synthetischen Polymere ausgewählt sind aus der Gruppe umfassend Polyacrylsäure und/oder Polyethylenimin.

14. Verfahren nach einem der Ansprüche 1 bis 13,
**dadurch gekennzeichnet, dass**
die erhaltenen Nanokapseln an ihrer Oberfläche modifiziert werden, vorzugsweise durch Polyethylenglycol, Proteine, Peptide oder Hormone, besonders bevorzugt durch Polyethylenglycol.

15. Nanokapseln hergestellt gemäß einem oder mehreren der Ansprüche 1 bis 14.

16. Nanokapseln mit einem Durchmesser von 50 nm bis 10µm,
**dadurch gekennzeichnet, dass**
ihre Hüllschicht aus mindestens zwei verschiedenen, miteinander kovalent vernetzten Polymeren P1 und P2 besteht.

17. Verwendung von Nanokapseln, hergestellt nach einem oder mehreren der Ansprüche 1 bis 14 zur Herstellung von pharmazeutischen Zubereitungen zur Applikation von Wirkstoffen.

18. Verwendung von Nanokapseln, hergestellt nach einem oder mehreren der Ansprüche 1 bis 14 zur biochemischen Diagnostik.

## Claims

1. A method of producing nanocapsules having a diameter of from 50 nm to 10 µm,
**characterized in that**
liposomes are produced which are coated with a polymer P1 by binding the polymer P1 covalently to the liposome surface in an aqueous solution, and the coated polymer P1 then is covalently crosslinked in an aqueous solution with a polymer P2 which is different from polymer P1, and additional polymer layers are optionally coated by crosslinking.

2. The method according to claim 1,
**characterized in that**
the liposomes are dissolved subsequent to crosslinking the polymers, preferably by leaching with a detergent.

3. The method according to claim 1 or 2,
**characterized in that**
liposomes are used as starting material which carry biologically active compounds or compounds of a detection system, which compounds remain in the nanocapsules when performing the method.

4. The method according to one of claims 1 to 3,
**characterized in that**
those polymers are used as water-soluble polymers P1 and P2 which have amino, carboxyl, thiol, hydrazo, hydroxy, acidic hydrogen, aldehyde and/or active ester groups or combinations of these groups as functional groups, and which do not themselves undergo formation of micellar or vesicular structures.

5. The method according to one of claims 1 to 4,
**characterized in that**
auxiliary agents are used to crosslink polymer P1 with the liposomes or polymer P1 with polymer P2.

6. The method according to claim 5,
**characterized in that**
isothiocyanates, isocyanates, acylazides, N-hydroxysuccinimide esters, sulfonyl chlorides, aldehydes, epoxides, carbonates, imidoesters, carbodiimides, anhydrides, haloacetyls, alkyl halides, maleimides, aziridines, pyridyldisulfides, diazoalkanes, diazoacetyls, carbonyldiimidazoles, N-hydroxysuccinimidylchloroformiates, or compounds containing these functional groups in suitable combinations are used as auxiliary agents.

7. The method according to one of claims 1 to 3,
**characterized in that**
the water-soluble polymers P1 or P2 have chelating or chelate-binding properties.

8. The method according to one of claims 1 to 6,
**characterized in that**
the polymers P1 and/or P2 are proteins.

9. The method according to one of claims 1 to 6
**characterized in that**
the proteins are selected from the group comprising of albumine, collagene, hemoglobine and/or fibrinogene.

10. The method according to one of claims 1 to 6,
**characterized in that**
the polymers P1 and/or P2 are carbohydrates.

11. The method according to one of claims 1 to 6
**characterized in that**
the carbohydrates are selected from the group comprising of alginate, heparine, pectine, chitosane, and/or hyaluron acid.

12. The method according to any of claims 1 to 6,
**characterized in that**
the water-soluble polymers P1 and/or P2 are synthetic polymers.

13. The method according to one of claims 1 to 6,
**characterized in that**
the synthetic polymers are selected from the group comprising of polyacryl acid and/or polyethylenimine.

14. The method according to any of claims 1 to 13,
**characterized in that**
the nanocapsules obtained are modified at their surface, preferably using poly(ethylene glycol), proteins, peptides, or hormones, with poly(ethylene glycol) being particularly preferred.

15. Nanocapsules, produced according to one or more of claims 1 to 14.

16. Nanocapsules having a diameter of from 50 nm to 10 µm,
**characterized in that**
the coat layer thereof is comprised of at least two different polymers P1 and P2 crosslinked with each other.

17. Use of the nanocapsules according to one or more of claims 1 to 14 in the production of pharmaceutical formulations used in the application of active substances.

18. Use of the nanocapsules according to one or more of claims 1 to 14 in biochemical diagnostics.

## Revendications

1. Procédé de préparation de nanocapsules d'un diamètre de 50 nm à 10 µm,
**caractérisé en ce**
**qu'**on prépare des liposomes, on recouvre ceux-ci avec un polymère P1, en liant de manière covalente le polymère P1 en solution aqueuse à la surface des liposomes, puis on réticule par liaison covalente le polymère appliqué P1 avec un polymère P2 différent de P1 en solution aqueuse et on applique éventuellement encore d'autres couches de polymère par réticulation.

2. Procédé selon la revendication 1,
**caractérisé en ce**
**qu'**on désintègre les liposomes après réticulation des polymères, de préférence par rinçage avec un détergent.

3. Procédé selon la revendication 1 ou 2,
**caractérisé en ce**
**qu'**on part de liposomes, qui portent des composés biologiquement actifs ou des composés d'un système de détection, lesquels demeurent dans les nanocapsules lors de la mise en oeuvre du procédé.

4. Procédé selon l'une des revendications 1 à 3,
**caractérisé en ce**
**qu'**en tant que polymères hydrosolubles P1 et P2, on utilise des polymères qui comportent à titre de groupements fonctionnels des groupements amino, carboxyle, thiol, hydrazo, hydroxy, hydroazoture, aldéhyde et/ou ester actif ou des combinaisons de ces groupements et qui ne forment pas par eux-mêmes des structures micellaires ou vésiculaires.

5. Procédé selon l'une des revendications 1 à 4,
**caractérisé en ce**
**qu'**on réticule le polymère P1 avec les liposomes ou le polymère P1 avec le polymère P2 au moyen d'agents auxiliaires.

6. Procédé selon la revendication 5,
**caractérisé en ce**
**qu'**en tant qu'agents auxiliaires, on utilise des isothiocyanates, des isocyanates, des acyl azides, des N-hydroxysuccinimide esters, des sulfonyl chlorures, des aldéhydes, des époxydes, des carbonates, des imidoesters, des carbodiimides, des anhydrides, des haloacétyles, des alkylhalogénures, des maléimides, des aziridines, des pyridiyldisulfures, des diazoalcanes, des diazoacétyles, des carboyldiimidazoles, des N-hydroxysuccinimidylchloroformiates ou des composés, qui contiennent ces groupements fonctionnels en combinaison appropriée.

7. Procédé selon l'une des revendications 1 à 3,
**caractérisé en ce que**
les polymères hydrosolubles P1 ou P2 présentent des propriétés chélatantes.

8. Procédé selon l'une des revendications 1 à 6,
**caractérisé en ce que**
les polymères P1 et/ou P2 sont des protéines.

9. Procédé selon l'une des revendications 1 à 6,
**caractérisé en ce que**
les protéines sont choisies dans le groupe comprenant l'albumine, le collagène, l'hémoglobine et/ou le fibrinogène.

10. Procédé selon l'une des revendications 1 à 6,
**caractérisé en ce que**
les polymères P1 et/ou P2 sont des hydrates de carbone.

11. Procédé selon l'une des revendications 1 à 6,
**caractérisé en ce que**
les hydrates de carbone sont choisis parmi le groupe comprenant l'alginate, l'héparine, la pectine, le chitosane et/ou l'acide hyaluronique.

12. Procédé selon l'une des revendications 1 à 6,
**caractérisé en ce que**
les polymères hydrosolubles P1 et/ou P2 sont des polymères synthétiques.

13. Procédé selon l'une des revendications 1 à 6,
**caractérisé en ce que**
les polymères synthétiques sont choisis parmi le groupe comprenant l'acide polyacrylique et/ou le polyéthylène imine.

14. Procédé selon l'une des revendications 1 à 13,
**caractérisé en ce**
**qu'**on modifie les nanocapsules obtenues sur leur surface, de préférence au moyen de polyéthylène glycol, de protéines, de peptides ou d'hormones, de manière plus préférée au moyen de polyéthylène glycol.

15. Nanocapsules préparées selon l'une ou plusieurs des revendications 1 à 14.

16. Nanocapsules ayant un diamètre de 50 nm à 10 µm,
**caractérisées en ce que**
leur couche d'enveloppe est constituée d'au moins deux polymères différents P1 et P2 réticulés par liaison covalente.

17. Utilisation de nanocapsules fabriquées selon l'une ou plusieurs des revendications 1 à 14 pour préparer des formulations pharmaceutiques destinées à l'administration de principes actifs.

18. Utilisation de nanocapsules fabriquées selon l'une ou plusieurs des revendications 1 à 14 pour le diagnostic biochimique.
